# EUROPEAN PATENT SPECIFICATION

(11) **EP 1 691 773 B1**
(45) Date of publication and mention of the grant of the patent: **28.05.2008**
(21) Application number: 04765493.4
(22) Date of filing: 21.09.2004
(51) Int. Cl.: A61K 8/18

(54) **A DENTIFRICE COMPOSITION COMPRISING A SOLUBLE CALCIUM SEQUESTERING AGENT**
ZAHNPASTENZUSAMMENSETZUNG MIT EINEM LÖSLICHEN CALCIUMSEQUESTRIERMITTEL
UNE COMPOSITION POUR DENTIFRICE COMPRENANT UN AGENT SOLUBLE DECALCIUM SEQUESTRANT

(30) Priority: 23.09.2003 GB 0322296
(43) Date of publication of application: 23.08.2006
(73) Proprietor: GLAXO GROUP LIMITED, Greenford, Middlesex UB6 ONN (GB)
(72) Inventor: CREETH, Jonathan Edward, GlaxoSmithKline, Weybridge Surrey KT13 0DE (GB)
(74) Representative: Reeves, Julie Frances
(86) International application number: PCT/EP2004/010629
(87) International publication number: WO 2005/027858

(56) References cited:
- EP-A- 0 002 184
- EP-A- 0 045 826
- EP-A- 0 428 494
- WO-A-95/17158
- GB-A- 2 137 494
- US-A- 4 634 589
- US-A- 5 589 160
- US-A- 5 964 937
- US-A1- 2003 133 882
- PATENT ABSTRACTS OF JAPAN vol. 015, no. 285 (C-0851), 19 July 1991 (1991-07-19) & JP 03 099007 A (SHINANEN NEW CERAMIC:KK), 24 April 1991 (1991-04-24)

## Description

The present invention relates to dentifrice compositions, in particular compositions comprising a fluoride source and a soluble calcium sequestering agent that is not an oxidising agent, for cleaning natural teeth and dentures. Such compositions show excellent cleaning properties whilst at the same time low abrasion characteristics.

Dentifrices have been used for more than 2000 years and the principal purpose of a dentifrice has always been the removal of surface deposits from the teeth. It is known that removal of surface deposits from the teeth can be achieved through the use of either a chemical or mechanical cleaning agent. An example of a chemical cleaning agent is a calcium-sequestering agent, for example a polyphosphate salt, such as tripolyphosphate, and the mechanical agents are the abrasive materials, e.g. precipitated silica or calcium carbonate. An example of an abrasive cleaning agent is amorphous hydrated silica. Bleaching agents are sometimes included in dentifrice compositions to decolourise tooth stains. This will not however remove the stain effectively and, furthermore, effective bleaching agents can damage the oral tissues and are difficult to formulate successfully. Hydrogen peroxide is a well-known bleaching agent but with peroxide-containing dentifrices the lifetime of peroxide in the oral cavity is very short. Human plaque contains a significant level of the peroxide-degrading enzyme catalase, which means that it is very difficult to maintain an effective level of peroxide on the tooth surface for sufficient time for stain to be bleached. Use of more aggressive bleaching agents than peroxides however bring concerns over tissue damage.

Traditional dentifrice formulations contain a number of specific components for example abrasive agents, humectants, fluoride sources, binders, anti plaque agents, dyes, flavours, preservatives, water and other optional ingredients. Fluoride is an important aid to oral health, particularly for the health of the tooth's enamel. The effective removal of surface deposited stains from the teeth or a dental prosthesis is always associated with substantially abrasive formulations. It is however recognised that dentifrice compositions containing abrasive materials can damage the tooth surface as well as the surface of dental prosthetics so it is very important that a dentifrice formulation should provide effective cleaning without subjecting the tooth or prosthetic to excessive abrasion.

WO-A-95/17158 describes and claims a composition for reducing or removing surface deposited stains from natural teeth or dental prostheses comprising a dentally acceptable preparation comprising 5 to 15% by weight of a water soluble alkali metal tripolyphosphate. All dentifrice formulation examples shown in WO-A-95/17158 include conventional dental abrasives, for example abrasive silica, is disclosed in the range 5-80 wt.%.

The established method for determining the abrasivity of a dentifrice formulation is by measuring the Relative Dentine Abrasivity (RDA) (Hefferen, JJ. A laboratory method for measuring dentifrice abrasivity. *J*. *Dent. Res.* **55** 563-573,1976.). This assay measures loss of dentine due to extended brushing with a 25:40 w/w slurry of test toothpaste from prepared samples of human dentine. The dentine samples are irradiated to generate ³²P in the mineral. The assay measures radioactivity in the supernatant after brushing, relative to radioactivity liberated by brushing with a standard slurry of calcium pyrophosphate.

Cleaning performance is closely linked to stain removal and can be measured in various ways. There is a very useful published in-vitro stain removal (NSR) assay (Layer TM, McConville PS and Wicks MA. Stain removal efficacy of whitening toothpastes - in vitro studies. J. Dent. Res. **79**: 216 abstract 581, 2000) that is used in the present invention. This assay aims to maximise the relevance of the assay to the *in vivo* situation by using untreated naturally stained bovine teeth as substrate. The teeth are brushed for an extended period with a 1:3 slurry of test toothpaste in water. Stain removal is quantified using a chromameter. Performance may be measured relative to a Control dentifrice formulation containing 14% Zeodent 113^{™} abrasive silica (available from J.M. Huber Corporation) in a conventional base containing water, sorbitol, glycerin, PEG, flavour, SLS, sodium saccharin, Xanthum gum and sodium fluoride. IVSR is also known as the natural extrinsic stain removal assay or NESR and is referred to as such in some publications.

Cleaning efficiency may therefore be described as the ratio of the IVSR to the RDA.

IVSR values that are quoted herewith are relative to a control formulation that has been assigned an arbitrary value of 100.

EP 0 835 223B discloses an amorphous silica that has a low abrasivity value which, when incorporated into a dentifrice composition, maintains good cleaning characteristics. This publication however considers the abrasive silica as the only cleaning material of the composition and describes the amorphous silica in terms of its physical properties. Accordingly EP 0 835 223B claims an amorphous silica characterized by: an RDA value of between 30 and 70, an oil absorption capacity of between 100 and 155cm³/100g, and a BET surface area of up to 200m²/g.

From the literature it appears that many formulations that are dependent for their cleaning properties upon their abrasive content are described and claimed in terms of the physical properties of the abrasive material. For example EP-A-0 396 460 describes in Example 1 an amorphous silica in terms of BET surface area, oil uptake, pore volume, pH, refractive index and translucence.

EP-A-0 002 184 discloses the use of a sodium polyphosphate in fine granulate form for tooth cleaning, either by itself or in combination with a commercial toothpaste formulation, referring to the abrasive effect of the sodium polyphosphate and intensification of tooth cleaning by this material without damaging the substance of the teeth.

A highly effective dentifrice formulation without the traditional amounts of abrasive material has not been disclosed or described in the prior art. By the term "highly effective dentifrice" is meant to refer to a dentifrice that satisfactorily removes stain from natural teeth or prosthetics whilst at the same time not damaging the dentine, enamel or prosthetic due to excessive abrasivity.

It is an object of the present invention to provide a dentifrice composition, for natural teeth and dentures, which addresses one or more of the problems mentioned above.

Accordingly, the present invention provides a dentifrice composition comprising a soluble calcium sequestering agent that is not an oxidising agent, wherein the composition has an RDA value of below 30 and an IVSR value greater than 50 (when compared to a Control) and an orally acceptable vehicle, wherein the calcium sequestering agent is present in a proportion of 1-20 wt % and the abrasive is present in a proportion of 0-5 wt % of the composition.

Preferably the composition is a non-gel toothpaste being a viscous extrudable fluid which can be provided in a collapsible container e.g. a tube or a pump and extruded therefrom onto a toothbrush for use.

Preferably the RDA value is below 25, preferably below 20, and most preferably as low as possible, e.g. so that the composition is substantially non-abrasive. Preferably the NSR value is up to 250, preferably 200 and more preferably greater than 100, for example being in the range 75-150, e.g. 75-120.

The soluble calcium-sequestering agent may be a calcium-chelating agent. Suitable soluble calcium sequestering agents include:

Polyphosphate salts (also known as condensed phosphate salts) according to formula: M⁺n₊₂[PₙO₃ₙ₊₁], where n>1, M = alkali metal, hydrogen ion or ammonium ion. These include: Pyrophosphates, for example alkali metal salts of pyrophosphate, and pyrophosphate salts in which hydrogen ion and/or ammonium ion may partially substitute for the alkali metal ions. Examples of these are:

| | |
|---|---|
| Na₄P₂O₇ | Tetrasodium pyrophosphate |
| Na₂H₂P₂O₇ | Disodium dihydrogen pyrophosphate |
| K₄P₂O₇ | Tetrapotassium pyrophosphate |
| K₂H₂P₂0₇ | Dipotassium dihydrogen pyrophosphate |
| Na₂K₂P₂O₇ | Dipotassium disodium pyrophosphate |

Tripolyphosphates, for example alkali and mixed alkali metal salts of tripolyphosphate, and tripolyphosphate salts in which hydrogen ion and/or ammonium ion may partially substitute for the alkali metal ions. Examples are:

| | |
|---|---|
| Na₅P₃O₁₀ | Pentasodium tripolyphosphate |
| K₅P₃O₁₀ | Pentapotassium tripolyphosphate |

Higher polyphosphate salts such as sodium and potassium tetraphosphates, and hexametaphosphate salts, also known as 'glassy phosphates' or 'polypyrophosphates'. Carboxylates, for example: alkali metal citrate salts, which may be partially substituted with hydrogen ion or ammonium ion, alkali metal acetate, lactate, tartrate and malate salts, which may be partially substituted with hydrogen ion or ammonium ion. Alkali metal salts of ethylenediaminetetraacetic acid (EDTA), which may be partially substituted with hydrogen ion or ammonium ion and editronic acid.

Two or more of the above-mentioned calcium sequestering agents may be used in combination in the composition.

A preferred soluble calcium-sequestering agent is pentasodium tripolyphosphate, often referred to as sodium tripolyphosphate.

When high levels of soluble calcium-sequestering agents are used in a formulation, there is often a certain amount of undissolved solids in the formulation. Any undissolved calcium-sequestering agent in compositions of the present invention will thus during use dissolve rapidly and provide a cleaning effect in its dissolved state.

The soluble calcium-sequestering agent, such as sodium tripolyphosphate, may be present in a proportion 1-20 wt %, preferably 2-15 wt%, more preferably 5-15 wt% of the dentifrice composition. By using a proportion of the calcium-sequestering agent in the composition below the solubility limit thereof a gel or liquid compositions may be provided in which the calcium sequestering agent is in solution, so that the gel or liquid may include no undissolved solid particles, and may be a clear gel or liquid.

The composition may include a fluoride ion source. Fluoride ion may stabilise polyphosphates in the mouth. For a composition for use in cleaning natural teeth the inclusion of a fluoride ion source is also desirable because of its caries protection activity, but for a composition for use in cleaning artificial dentures such activity is not necessary. The fluoride ion source may be provided by an alkali metal fluoride, preferably sodium fluoride, an alkali metal monofluorophosphate, stannous fluoride and the like. Preferably, however, the fluoride ion source is an alkali metal fluoride, most preferably sodium fluoride. The fluoride ion source serves in a known manner for caries protection. Preferably, the fluoride ion source will be used in an amount to provide an anti-caries effective amount and a phosphatase enzyme inhibiting amount, such as an amount sufficient to provide from about 25 ppm to about 3500 ppm, preferably about 1100 ppm, as fluoride ion. For example the formulation may contain 0.1- 0.5 wt % of an alkali metal fluoride such as sodium fluoride.

Preferably the pH of the composition is from 6 to 10.5, more preferably from 7 to 9.5. Typically the composition may contain up to 0.5 wt.% of sodium hydroxide to provide a suitable pH.

In compositions of the present invention i.e. those which can be extruded onto a toothbrush, the orally acceptable vehicle may be of a generally conventional composition e.g. comprising a thickening agent, a binding agent and a humectant. Preferred binding agents include for example natural and synthetic gums such as xanthan gums, carageenans, alginates, cellulose ethers and esters. Preferred humectants include glycerin, sorbitol, propylene glycol and polyethylene glycol. A preferred humectant system consists of glycerin, sorbitol and polyethylene glycol.

In addition, the orally acceptable vehicle may optionally comprise one or more surfactant, sweetening agent, flavouring agent, anticaries agent (in addition to the fluoride ion source), anti-plaque agent, anti-bacterial agent such as triclosan or cetyl pyridinium chloride, tooth desensitizing agent, colouring agents and pigment. Useful surfactants include the water-soluble salts of alkyl sulphates having from 10 to 18 carbon atoms in the alkyl moiety, such as sodium lauryl sulphate, but other anionic surfactants as well as non-ionic, zwitterionic, cationic and amphoteric surfactants may also be used.

If an aqueous orally acceptable vehicle is employed, a toothpaste composition of the present invention suitably contains from about 10 to about 80 wt % humectant such as sorbitol, glycerin, polyethylene glycol or xylitol; from about 0.25 to about 5 wt % detergent; from 0 to about 2 wt % sweetener and flavouring agents; together with water and an effective amount of binding and thickening agents, such as from about 0.1 to about 15 wt %, to provide the toothpaste of the invention with the desired stability and flow characteristics.

It is preferred to use a thickening silica as the thickening agent. So called "thickening silicas" are known silicas which have relatively little abrasive effect compared with known abrasive silicas such as Zeodent 113^{™}, but provide a thickening effect on the composition. Suitable thickening silicas are known and include those marketed by Huber under the tradename Zeodent, e.g. Zeodent 167, by Degussa AG under the trade name SIDENT®, e.g. SIDENT 22S®, and by Grace-Davison Chemical Division under the trade name SYLOBLANC®, e.g. SYLOBLANC 15®, respectively. For example the composition may contain up to ca. 20wt % of a thickening silica, typically 5-15 wt %.

The composition of the invention may contain an abrasive material, for example known types of "abrasive silica" commonly used in toothpaste compositions e.g. Zeodent 113^{™} as mentioned above. However to achieve the RDA below 30 it is preferred to include as little of this type of abrasive material as possible, preferably less than 5 wt.%, more preferably less than 2 wt.% abrasive, most preferably 0 wt.% abrasive material, in addition to any mild abrasive effect produced by other solid particles in the composition, e.g. undissolved calcium sequestering agent and e.g. any thickening silica present. For the purposes of this invention an abrasive material may be defined as a material having an RDA of 30 or above. Larger amounts of silica may be incorporated, more typical of abrasive silica incorporation levels in toothpastes (up to e.g. 25%), if that silica has an RDA below 30.

Regarding the RDA values given in the preceding paragraph, it should be noted that the slurry conditions used to determine the RDA of an abrasive raw material differ from those used to determine the RDA of a formulated toothpaste. The slurry conditions for a raw material are 10g abrasive plus 50ml of a 0.5% carboxymethyl cellulose slurry in 10% glycerin, whereas the slurry conditions for a formulated toothpaste are 25g toothpaste plus 40ml water. Care should therefore be taken when comparing RDA figures for abrasives as raw materials with RDA figures for fully formulated toothpastes.

Therefore a preferred dentifrice composition of this invention comprises a soluble calcium sequestering agent that is not an oxidising agent, wherein the composition has an RDA value of below 30 and an NSR value greater than 50 (when compared to a Control) and an orally acceptable vehicle, wherein the calcium sequestering agent is present in a proportion 1-20 wt %, preferably 5-10 wt % of the composition, with 0 - 5 wt %, preferably 0 wt % of an abrasive, typically an abrasive silica.

The dentifrice composition may be presented as either a single or dual phase composition.

Suitably the composition is in the form of a conventional toothpaste-type composition that can be squeezed from a collapsible tube. It is also suitable for dispensing from a pressurised aerosol container.

Toothpaste-type compositions according to the present invention may be prepared by admixing according to conventional practice the calcium sequestering agent, and the fluoride ion source if present, with the orally acceptable dental vehicle, which may be anhydrous but is preferably an aqueous orally acceptable dental vehicle, to form a storage-stable semi-solid extrudable material useful as a dentifrice.

The composition of the invention will now be described by way of non-limiting examples only.

**Table 1. Examples of the present invention using different calcium sequestering systems**

| **Ingredient** | **Example** | | | | | | |
|---|---|---|---|---|---|---|---|
| | **Control** | **1** | **2** | **3** | **4** | **5** | **6** |
| Glycerin, 98% min | 10.00 | 11.20 | 11.20 | 11.20 | 11.20 | 11.20 | 11.20 |
| Sorbitol, 70% soln. | 26.00 | 29.11 | 29.11 | 29.11 | 29.11 | 29.11 | 29.11 |
| Peg 6 | 3.00 | 3.00 | 3.00 | 3.00 | 3.00 | 3.00 | 3.00 |
| Xanthan gum | 1.20 | 0.70 | 0.70 | 0.70 | 0.70 | 0.70 | 0.70 |
| Flavour | 1.00 | 1.00 | 1.00 | 1.00 | 1.00 | 1.00 | 1.00 |
| Soluble saccharin | 0.21 | 0.21 | 0.21 | 0.21 | 0.21 | 0.21 | 0.21 |
| Sodium lauryl sulphate | 1.15 | 1.15 | 1.15 | 1.15 | 1.15 | 1.15 | 1.15 |
| Sodium tripolyphosphate | 0.00 | 10.00 | 0.00 | 10.00 | 0.00 | 4.00 | 0.00 |
| Tetra sodium pyrophosphate | 0.00 | 0.00 | 1.80 | 0.00 | 0.00 | 0.90 | 0.00 |
| Tetrapotassium pyrophosphate | 0.00 | 0.00 | 4.00 | 0.00 | 0.00 | 2.00 | 0.00 |
| Sodium trimetaphosphate | 0.00 | 0.00 | 0.00 | 5.00 | 0.00 | 0.00 | 0.00 |
| Sodium Hexametaphosphate | 0.00 | 0.00 | 0.00 | 0.00 | 7.00 | 0.00 | 0.00 |
| Trisodium citrate | 0.00 | 0.00 | 0.00 | 0.00 | 0.00 | 0.00 | 5.00 |
| Sodium hydroxide | 0.00 | 0.00 | 0.25 | 0.25 | 0.25 | 0.25 | 0.00 |
| Sodium fluoride | 0.24 | 0.24 | 0.24 | 0.24 | 0.24 | 0.24 | 0.24 |
| Titanium dioxide | 1.00 | 1.45 | 1.00 | 1.00 | 1.00 | 1.00 | 1.00 |
| Abrasive silica Zeodent 113 | 14.00 | 0.00 | 0.00 | 0.00 | 0.00 | 0.00 | 0.00 |
| Thickening silica | 9.00 | 13.50 | 13.50 | 13.50 | 13.50 | 13.50 | 13.50 |
| De-ionised water | 33.20 | 28.44 | 32.84 | 23.64 | 31.64 | 31.74 | 33.89 |
| Total | 100.00 | 100.00 | 100.00 | 100.00 | 100.00 | 100.00 | 100.00 |

Various embodiments of the invention may be prepared.

Example 1 is a formulation in which the calcium-sequestering chemical cleaning system is sodium tripolyphosphate (STP). Thickening silica is increased to account for the lower level of solids in the formulation. Other polyphosphates may be used as chemical cleaning agent and/or anticaries agent.

Example 2 is an equivalent formulation using mixed pyrophosphate salts instead of STP. Sodium hydroxide has been added to increase the pH and thereby increase the stability of the polyphosphate component.

Example 3 uses trimetaphosphate, and example 4 sodium hexametaphosphate.

Example 5 shows that mixed calcium-sequestration systems may be used, this embodiment combining different polyphosphate salts.

Example 6 uses an effective amount of a polycarboxylate, trisodium citrate, as a calcium-sequestering agent.

**Table 2. Examples of the present invention**

| **Ingredient** | **Example** | | | | | |
|---|---|---|---|---|---|---|
| | **7** | **8** | **9** | **10** | **11** | **12** |
| Glycerin, 98% min | 11.20 | 0.00 | 13.00 | 11.20 | 11.20 | 10.00 |
| Sorbitol, 70% soln. | 29.11 | 65.00 | 35.00 | 29.11 | 29.11 | 26.00 |
| Peg 6 | 3.00 | 3.00 | 3.00 | 3.00 | 3.00 | 3.00 |
| Xanthan gum | 1.50 | 0.50 | 1.80 | 0.70 | 0.40 | 0.70 |
| Flavour | 1.00 | 1.00 | 1.00 | 1.00 | 1.00 | 1.00 |
| Soluble saccharin | 0.21 | 0.15 | 0.21 | 0.21 | 0.21 | 0.21 |
| Sodium lauryl sulphate | 1.15 | 1.15 | 1.15 | 1.15 | 1.15 | 0.00 |
| Tegobetaine | 0.00 | 0.00 | 0.00 | 0.00 | 0.00 | 2.00 |
| Sodium tripolyphosphate | 10.00 | 1.00 | 10.00 | 10.00 | 10.00 | 10.00 |
| Tetra sodium pyrophosphate | 0.00 | 0.90 | 0.00 | 0.00 | 0.00 | 0.00 |
| Tetrapotassium pyrophosphate | 0.00 | 2.00 | 0.00 | 0.00 | 0.00 | 0.00 |
| Trisodium citrate | 0.00 | 1.00 | 0.00 | 0.00 | 0.00 | 0.00 |
| Sodium hydroxide | 0.00 | 0.30 | 0.00 | 0.30 | 0.00 | 0.00 |
| Sodium fluoride | 0.24 | 0.24 | 0.24 | 0.24 | 0.24 | 0.24 |
| Titanium dioxide | 1.00 | 0.00 | 1.00 | 1.00 | 1.00 | 1.00 |
| Abrasive silica Zeodent 113 | 0.00 | 0.00 | 0.00 | 2.00 | 0.00 | 0.00 |
| Polypropylene beads | 0.00 | 0.00 | 0.00 | 0.00 | 0.00 | 15.00 |
| Thickening silica | 6.50 | 11.00 | 0.00 | 13.50 | 9.00 | 6.50 |
| De-ionised water | 35.09 | 12.76 | 33.60 | 26.59 | 33.69 | 24.35 |
| Total | 100.00 | 100.00 | 100.00 | 100.00 | 100.00 | 100.00 |

Example 7 shows that the thickening silica need not be increased to compensate for the lack of abrasive silica. In this example the level of thickening silica is the same as in the formulation containing the normal level of abrasive (6.5%). However, the gum level is raised to maintain adequate viscosity.

Example 8 shows that clear gels may be prepared according to the present invention. In this case it is important to keep the level of certain calcium-sequestering salts below or only slightly above their limit of solubility, so a combination of different salts is used. This example also shows that polyphosphates may be combined with carboxylate calcium-sequestering agents.

Example 9 shows that silica may be omitted from the formulation altogether whilst still maintaining good cleaning from calcium-sequestering agents.

Example 10 shows that low levels of abrasive silica may be included in the formulations of the present invention. Provided the final RDA value is below 30, it is found that the remarkable cleaning efficiency of the invention is retained.

Example 11 shows a low-viscosity liquid toothpaste formulation.

Example 12 shows a formulation with zwitterionic surfactant, which may be employed to minimise any possible irritation to oral tissues.

**Table 3. Examples of the present invention with different therapeutic activities**

| **Ingredient** | **Example number** | | | | |
|---|---|---|---|---|---|
| | **13** | **14** | **15** | **16** | **17** |
| Glycerin, 98% min | 11.20 | 0.00 | 0.00 | 11.20 | 22.00 |
| Sorbitol, 70% soln. | 29.11 | 45.00 | 29.11 | 29.11 | 28.00 |
| Xylitol | 0.00 | 0.00 | 10.00 | 0.00 | 0.00 |
| Peg 6 | 3.00 | 2.00 | 3.00 | 3.00 | 2.50 |
| Xanthan gum | 0.70 | 0.90 | 0.70 | 0.70 | 0.25 |
| Flavour | 0.80 | 1.20 | 1.00 | 1.00 | 1.70 |
| Soluble saccharin | 0.21 | 0.21 | 0.21 | 0.21 | 0.30 |
| Sodium lauryl sulphate | 0.80 | 1.50 | 1.15 | 0.00 | 1.50 |
| Triclosan | 0.00 | 0.30 | 0.00 | 0.00 | 0.00 |
| F127* | 0.00 | 0.00 | 0.00 | 2.00 | 0.00 |
| Sodium tripolyphosphate | 5.00 | 10.00 | 4.00 | 10.00 | 5.00 |
| Tetra sodium pyrophosphate | 0.00 | 0.00 | 1.80 | 0.00 | 0.00 |
| Casein phosphopeptide | 0.00 | 0.00 | 2.00 | 0.00 | 0.00 |
| Tetrapotassium pyrophosphate | 0.00 | 0.00 | 4.00 | 0.00 | 0.00 |
| Potassium nitrate | 5.00 | 0.00 | 0.00 | 0.00 | 0.00 |
| Lactoperoxidase | 0.00 | 0.00 | 0.00 | 0.20 | 0.00 |
| Glucose oxidase | 0.00 | 0.00 | 0.00 | 0.20 | 0.00 |
| Sodium hydroxide | 0.35 | 0.00 | 0.00 | 0.00 | 0.00 |
| Sodium fluoride | 0.24 | 0.24 | 0.55 | 0.24 | 0.24 |
| Titanium dioxide | 1.00 | 1.00 | 1.00 | 1.00 | 0.00 |
| Thickening silica | 13.50 | 12.50 | 13.50 | 13.50 | 5.50 |
| Butane | 0.00 | 0.00 | 0.00 | 0.00 | 3.00 |
| Dimethyl ether | 0.00 | 0.00 | 0.00 | 0.00 | 2.00 |
| De-ionised water | 29.09 | 25.15 | 27.98 | 27.64 | 28.01 |
| Total | 100.00 | 100.00 | 100.00 | 100.00 | 100.00 |

| | | | | | |
|---|---|---|---|---|---|
| *Ethylene oxide-propylene oxide block copolymer. | | | | | |

Example 13 is a formulation according to the present invention containing therapeutic amounts of potassium nitrate to relieve the pain of sensitive teeth.

Example 14 is an anti-gingivitis formulation according to the present invention based on inclusion of Triclosan.

Example 15 is a formulation according to the present invention designed to provide highly effective anticavity protection, containing 2500ppm fluoride, xylitol and casein phosphopeptide.

Example 16 is a formulation according to the present invention containing active enzymes to control plaque bacteria, using non-ionic surfactant to maximise the stability of the biological molecules.

Example 17 is a formulation according to the present invention suitable for dispensing from a pressurised aerosol container.

**Table 4. RDA data for a typical abrasive silica + STP formulation.**

| Abrasive level (%) | STP level (%) | RDA | Std error |
|---|---|---|---|
| 14 | 10 | 86.70 | +/-3.16 |

The RDA of a 14% Zeodent 113 abrasive silica formulation as shown in Table 4 was tested. The RDA value is above that of a formulation of the invention.

### Cleaning efficacy and abrasivity testing.

The cleaning efficacy of a formulation given in Example 1 was tested. This formulation was tested against the standard abrasive-containing Control toothpaste formulation (see Table 1), with water as a negative control, using the method of Layer *et al.* The formulations were also sent to the Oral Health Research Institute, University of Indiana for RDA testing. The values for the stain removal and abrasivity are given in Table 4 below:

**Table 5. IVSR cleaning and abrasivity data for formulation Example 1**

| Formulation | Silica level | STP level | RDA | IVSR cleaning |
|---|---|---|---|---|
| Control | 14 | 0 | 50.69 ± 2.97 | 100 ± 9.42 |
| Example 1 | 0 | 10 | 9.99 ± 0.47 | 115.51 ± 10.78 |
| Water | - | - | - | -10.82 ± 14.26 |

As can be seen from Table 4, the abrasivity of the formulation according to the present invention Example 1, was extremely low (RDA=9.99). The control paste gave an abrasivity in the expected range for formulations of this type. However, the cleaning value of the prototype formulation was 115.5 of the abrasive-containing control, demonstrating extremely effective cleaning performance. The table also shows that brushing with water does not remove stain from this substrate.

## Claims

1. A dentifrice composition comprising a soluble calcium sequestering agent that is not an oxidising agent, wherein the composition has an RDA value of below 30 and an IVSR value greater than 50 (when compared to a Control), and an orally acceptable vehicle, wherein the calcium sequestering agent is present in a proportion of 1-20 wt % and an abrasive is present in a proportion of 0-5 wt % of the composition.

2. A dentifrice composition according to claim 1, further comprising an abrasive silica that has an RDA below 30.

3. A dentifrice composition according to claim 2, wherein the RDA value is below 20 and the IVSR value is up to 250.

4. A dentifrice composition according to claim 3, wherein the calcium sequestering agent is present in an amount of from 2-15 wt % of the composition.

5. A dentifrice composition according to claim 4, wherein the soluble calcium sequestering agent is a calcium-chelating agent.

6. A dentifrice composition according to claim 5, wherein the calcium-chelating agent is a polyphosphate or pyrophosphate salt.

7. A dentifrice composition according to claim 6, wherein the pyrophosphate salt is pentasodium tripolyphosphate.

8. A dentifrice composition according to claim 7, wherein 0 wt% of the abrasive is present in the composition.

9. A dentifrice composition according to claim 8, wherein the IVSR value is measured relative to a Control dentifrice containing 14% Zeodent 113 abrasive silica in a conventional base containing water, sorbitol, glycerin, PEG, flavour, SLS, sodium saccharin, Xanthum gum and sodium fluoride.

10. A dentifrice composition according to any one of the above claims for use in treating natural teeth or a dental prosthetic.

11. A dentifrice composition according to any one of the above claims in the form of a viscous extrudable fluid which can be provided in a collapsible container.

## Patentansprüche

1. Zahnreinigungszusammensetzung, umfassend einen löslichen Calciumkomplexbildner, wobei es sich nicht um ein Oxidationsmittel handelt, wobei die Zusammensetzung einen RDA-Wert unterhalb von 30 und einen IVSR-Wert von mehr als 50 (im Vergleich zu einer Kontrolle) aufweist, und einen oral annehmbaren Träger, wobei der Calciumkomplexbildner in einem Anteil von 1 bis 20 Gew.% und ein Abriebmittel in einem Anteil von 0 bis 5 Gew.% der Zusammensetzung vorliegt.

2. Zahnreinigungszusammensetzung gemäss Anspruch 1, weiterhin umfassend ein Abriebsilica mit einem RDA-Wert unterhalb von 30.

3. Zahnreinigungszusammensetzung gemäss Anspruch 2, wobei der RDA-Wert unterhalb von 20 und der IVSR-Wert bei bis zu 250 liegt.

4. Zahnreinigungszusammensetzung gemäss Anspruch 3, wobei der Calciumkomplexbildner in einer Menge von 2 bis 15 Gew. der Zusammensetzung vorliegt.

5. Zahnreinigungszusammensetzung gemäss Anspruch 4, wobei der lösliche Calciumkomplexbildner ein Calciumchelatbildner ist.

6. Zahnreinigungszusammensetzung gemäss Anspruch 5, wobei der Calciumchelatbildner ein Polyphosphat- oder Pyrophosphatsalz ist.

7. Zahnreinigungszusammensetzung gemäss Anspruch 6, wobei das Pyrophosphatsalz Pentanatriumtripolyphosphat ist.

8. Zahnreinigungszusammensetzung gemäss Anspruch 7, wobei 0 Gew.% des Abriebmittels in der Zusammensetzung vorliegt.

9. Zahnreinigungszusammensetzung gemäss Anspruch 8, wobei der IVSR-Wert relativ zu einem Kontroll-Zahnreinigungsmittel, enthaltend 14 % Zeodent 113-Abriebsilica in einer konventionellen Basis, enthaltend Wasser, Sorbit, Glycerin, PEG, Geschmacksstoff, SLS, Natriumsaccharin, Xanthangummi und Natriumfluorid, gemessen wird.

10. Zahnreinigungszusammensetzung gemäss einem der obigen Ansprüche zur Verwendung bei der Behandlung natürlicher Zähne oder dentaler Prothesen.

11. Zahnreinigungszusammensetzung gemäss einem der obigen Ansprüche in Form eines viskosen extrudierbaren Fluids, das in einem kollabierbaren Behälter bereitgestellt werden kann.

## Revendications

1. Composition de dentifrice comprenant un agent soluble séquestrant de calcium qui n'est pas un agent oxydant, dans laquelle la composition a une valeur RDA inférieure à 30 et une valeur IVSR supérieure à 50 (par rapport à un contrôle) et un excipient oralement acceptable, dans lequel l'agent séquestrant de calcium est présent dans une proportion de 1-20% en poids et un abrasif est présent dans une proportion de 0-5% en poids de la composition.

2. Composition de dentifrice selon la revendication 1, comprenant en outre une silice abrasive ayant une RDA inférieure à 30.

3. Composition de dentifrice selon la revendication 2, dans laquelle la valeur RDA est inférieure à 20 et la valeur IVSR peut atteindre 250.

4. Composition de dentifrice selon la revendication 3, dans laquelle l'agent séquestrant de calcium est présent dans une quantité de 2-15% en poids de la composition.

5. Composition de dentifrice selon la revendication 4, dans laquelle l'agent séquestrant de calcium soluble est un agent chélatant de calcium.

6. Composition de dentifrice selon la revendication 5, dans laquelle l'agent chélatant de calcium est un polyphosphate ou un sel de pyrophosphate.

7. Composition de dentifrice selon la revendication 6, dans laquelle le sel de pyrophosphate est du tripolyphosphate de pentasodium.

8. Composition de dentifrice selon la revendication 7, dans laquelle 0% en poids de l'abrasif est présent dans la composition.

9. Composition de dentifrice selon la revendication 8, dans laquelle la valeur IVSR est mesurée relativement à un dentifrice de contrôle contenant 14% de silice abrasive Zeodent 113 dans une base conventionnelle contenant de l'eau, du sorbitol, de la glycérine, du PEG, un arôme, du SLS, de la saccharine de sodium, de la gomme de Xanthane et du fluorure de sodium.

10. Composition de dentifrice selon l'une quelconque des revendications précédentes à utiliser dans le traitement de dents naturelles ou d'une prothèse dentaire.

11. Composition de dentifrice selon l'une quelconque des revendications précédentes sous la forme d'un fluide extrudable visqueux qui peut être fourni dans un conteneur pliable.
